# EUROPEAN PATENT SPECIFICATION

(11) **EP 2 566 407 B1**
(45) Date of publication and mention of the grant of the patent: **26.03.2014**
(21) Application number: 11728071.9
(22) Date of filing: 03.05.2011
(51) Int. Cl.: A61B 17/34

(54) **SEAL DEVICE FOR TROCAR AND RELATED TROCAR**
DICHTUNGSVORRICHTUNG FÜR TROKAR UND ZUGEHÖRIGER TROKAR
DISPOSITIF D'ÉTANCHÉITÉ POUR TROCART ET TROCART ASSOCIÉ

(30) Priority: 03.05.2010 IT RM20100207
(43) Date of publication of application: 13.03.2013
(73) Proprietor: AB Medica S.p.A., 20123 Milano (IT)
(72) Inventor: ORLANDI, Fabio, I-20123 Milano (IT)
(74) Representative: Spina, Alessandro
(86) International application number: PCT/IT2011/000136
(87) International publication number: WO 2011/138812

(56) References cited:
- EP-A1- 1 459 688
- US-A1- 2003 195 472
- US-A1- 2009 093 682

## Description

The present invention relates to a seal device for trocar, in particular for laparoscopic surgery, that allows in a manner that is reliable, versatile, simple, inexpensive, comfortable for the operator and safe for the patient to introduce within the patient's abdominal cavity surgical instruments having varying diameter, including optical devices, ensuring the hydraulic tightness around the surgical instrument inserted into the trocar.

The present invention further relates to a trocar comprising such a seal device.

It is known that laparoscopic surgery has been widely spread in recent years thanks to its reduced invasivity.

In order to perform an intervention with laparoscopic technique, an invasive surgical device called trocar, usually disposable sterile, is used that allows to make a small incision on the abdominal walls through which all the surgical instruments, such as scalpels, washing and/or suction cannulae, and optical devices, needed for the intervention, may be introduced within the abdominal cavity.

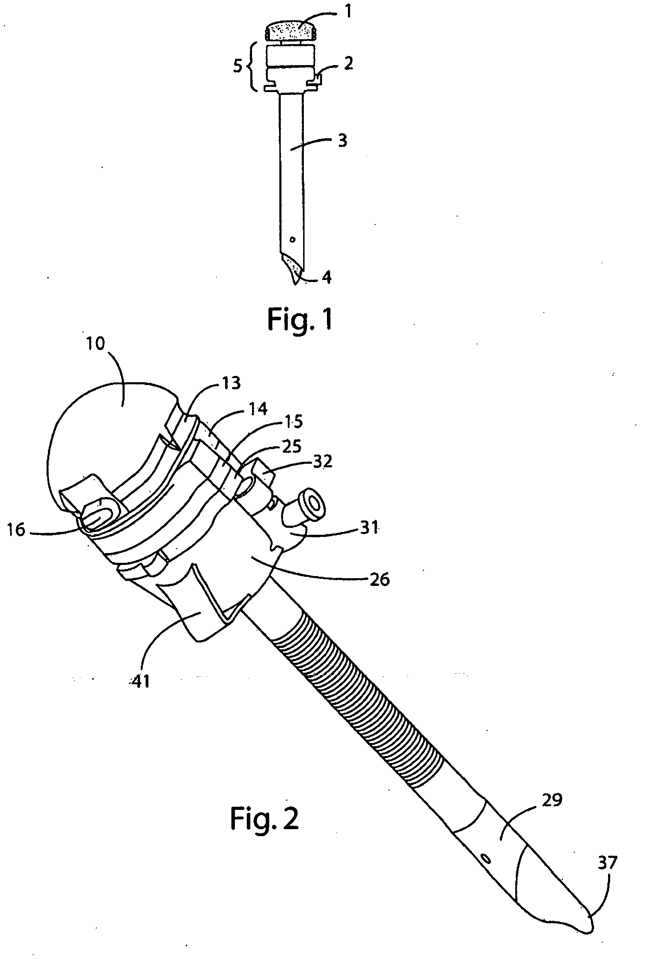

With reference to Figure 1, it may be observed that a trocar comprises a body 5 coupled (usually integrally) to a hollow cylindrical cannula 3 the distal end of which is flared, i.e. cut according to a plane sloped with respect to the plane orthogonal to the longitudinal axis of the cannula 3. A cap 1 is removably coupled to the body 5 and is provided with a cylindrical obturator ending, at its distal end, with a tip 4 capable to perforate tissues that, usually, is whistle shaped. When the cap 1 is coupled to the body 5, the obturator is inserted within the cannula 3 from which only the tip 4 protrudes, so that, when the surgeon handles the cap 1-body 5 assembly and presses the tip 4 against the abdominal walls, the tip cuts the tissues of the same walls introducing the distal portion of the cannula 3 in the abdominal cavity. At the end of the incision, the cap 1 and the obturator are removed for allowing the surgical instruments to be introduced, through the cannula 3 operating as passage channel, in the abdominal cavity. The body 5 is provided with an outer valve 2 for connection of external apparatuses for insufflation (e.g. for gas introduction) and irrigation, still through the cannula 3, with the abdominal cavity. The body 5 internally houses further valves (not shown in Figure 1) ensuring tightness of the cannula 3, even when surgical instruments are present.

In order to maintain the hydraulic tightness around the surgical instrument inserted into the trocar, some seal membranes and devices are known in prior art.

Document EP 1348386 discloses a conical reinforced membrane formed by a plurality of layers.

Document EP 1459688 discloses a seal device for trocar comprising two membranes having respective pluralities of flexible protecting elements partially :overlapping each other for forming a conical surface.

Document US 5385552 discloses a planar membrane made with a plurality of partially overlapping planar elements having semi-circular angular width.

Document US 4655752 discloses a conical membrane made with a sole monolayer conical element.

Document US 2009/093682 A1 discloses a seal device for a trocar, comprising a proximal truncated tapered element, of rigid elastic material, composed of a plurality of conical sectors,
a proximal membrane, of elastic material, centrally provided with a through hole, a distal truncated tapered element, of rigid elastic material, composed of one or more conical sectors,
a distal foam membrane, of soft elastic material, comprising a tapered surface and
a distal annular bracket, of rigid plastic material, the device further comprising coupling mechanical means coupling the proximal truncated element, the proximal membrane, the distal truncated element, said distal foam membrane and the distal annular bracket to a proximal housing, whereby the proximal truncated tapered element is inserted in the distal truncated tapered element that is in turn inserted in said distal foam membrane.

However, all prior art trocars suffer from some drawbacks, mainly due to the difficulty of efficiently maintaining the hydraulic tightness.

In this context, the solution proposed according to the present invention is introduced, allowing to overcome the aforementioned problems

It is therefore an object of the present invention to allow in a manner that is reliable, versatile, simple, inexpensive, comfortable for the operator and safe for the patient to introduce within the patient's abdominal cavity surgical instruments having varying diameter, including optical devices, ensuring the hydraulic tightness around the surgical instrument inserted into the trocar.

It is specific subject matter of this invention as claimed in claim 1, a seal device for trocar, characterised in that it comprises, in the following order:
- a proximal truncated conical element, of rigid elastic material, composed of a plurality of conical sectors,
- a proximal annular shaped membrane, of soft elastic material, centrally provided with a through hole,
- a distal truncated conical element, of rigid elastic material, composed of a plurality of conical sectors,
- a distal membrane with two or more truncated conical elements, of soft elastic material, overlapping each other, each one of which comprises a conical surface and
- a distal annular bracket, of rigid plastic material,
the device further comprising coupling mechanical means coupling the proximal truncated conical element, the proximal membrane, the distal truncated conical element, said two or more truncated conical elements of said distal membrane and the distal annular bracket to each other, whereby the proximal truncated conical element is inserted in the distal truncated conical element that is in turn inserted in said distal membrane.

Always according to the invention, the conical surface of each of said truncated conical elements of said distal membrane is provided with at least one linear slit aligned along a generating line of the same conical surface, said plurality of truncated conical elements being overlapped one another with the respective linear slits rotated from each other so as to be misaligned, preferably rotated from each other by an angle equal to the ratio between 360° and the number of said two or more truncated conical elements.

Still according to the invention, said two or more truncated conical elements of said distal membrane may be three in number.

Furthermore according to the invention, the proximal truncated conical element may be composed of at least two, preferably three, conical sectors and the distal truncated conical element may be composed of at least two, preferably three, conical sectors, the proximal truncated conical element being preferably inserted in the distal truncated conical element so that the respective conical sectors are rotated from each other so that separating spaces between said at least two conical sectors of the proximal truncated conical element are misaligned with respect to said at least two conical sectors of the proximal truncated conical element, the proximal truncated conical element and the distal truncated conical element being more preferably composed of a same number of respective conical sectors, rotated from each other by an angle still more preferably equal to the half of the ratio between 360° and said same number of conical sectors.

Always according to the invention, said coupling mechanical means may comprise at least one plurality of pins inserted in one or more corresponding pluralities of holes, said pins being preferably pressed in said holes and swaged, more preferably:
- the proximal truncated conical element comprising a proximal circular rim, to which said one or more conical sectors are connected, provided on a distal surface with a plurality of pins,
- the proximal membrane comprising a circular rim, delimiting the central through hole, provided with a plurality of through holes corresponding to said plurality of pins,
- the distal truncated conical element comprising a proximal circular rim, to which said one or more conical sectors are connected, provided with a plurality of through holes corresponding to said plurality of pins,
- each one of said two or more truncated conical elements comprising a proximal circular rim, to which the conical surface is connected, provided with a plurality of through holes corresponding to said plurality of pins,
- the distal annular bracket being provided with a plurality of holes, still more preferably through holes, corresponding to said plurality of pins.

Still according to the invention, the proximal membrane may comprise a circular rim, delimiting the central through hole, from which a projecting edge extends, a proximal end of the distal truncated conical element being housed in the proximal membrane in contact with a distal surface of the circular rim thereof and internally to the projecting edge of the latter, the proximal membrane having a shape with circular plan that is frontally slightly squeezed along a circle arc, preferably ranging from 90° to 120°.

Furthermore according to the invention, a distal end of the conical surface of at least one of said two or more truncated conical elements of said distal membrane may be linked to a cylindrical surface.

Always according to the invention, the device may be housed in a first and a second annular elements, coupled to each other to form a container of the device and having a central through hole, the first and second annular elements being preferably of polycarbonate, the first and second annular elements being more preferably glued to each other.

Still according to the invention:
- the proximal truncated conical element may be made of at least one material selected from the group comprising a thermoplastic elastomer, preferably Hytrel from DuPont®, and polycarbonate, and/or
- the proximal membrane may be made of at least one material selected from the group comprising silicone, laprene, mix of styrene butadiene rubber (SBR) and nitrile rubber (NBR), the material of the proximal membrane having preferably hardness equal to 40 shore A, and/or
- the distal truncated conical element may be made of thermoplastic elastomer, preferably Hytrel from DuPont®, and/or polycarbonate, and/or
- said two or more truncated conical elements of said distal membrane may be made of at least one material selected from the group comprising silicone, laprene, mix of styrene butadiene rubber (SBR) and nitrile rubber (NBR), the material of the proximal membrane having preferably hardness equal to 40 shore A, and/or
- the distal annular bracket may be made of at least one material selected from the group comprising a thermoplastic polymer, preferably acrylonitrile butadiene styrene or ABS, and polycarbonate.

It is still specific subject matter of this invention a trocar comprising a body, coupled to a hollow cylindrical cannula in which an obturator is insertable, and a top cap, having a top, that is removably coupled to the body, characterised in that the body comprises a seal device as previously described.

The self-centring seal device for trocar according to the invention allows to efficiently maintain the hydraulic tightness around the surgical instrument inserted into the trocar.

Moreover, the self-centring seal device according to the invention allows to ensure the hydraulic tightness for a wide variety of diameters of the surgical instruments, preferably ranging from 5 to 12mm.

Also, the self-centring seal device according to the invention can be easily manufactured, with extremely reduced production costs.

The present invention will be now described, by way of illustration and not by way of limitation, according to its preferred embodiments, by particularly referring to the Figures of the enclosed drawings, in which:
Figure 1 shows a schematic right side view of a trocar of the prior art;
Figure 2 shows a perspective view of a preferred embodiment of the trocar provided with the seal device according to the invention;
Figure 3 shows a perspective exploded view of the trocar of Figure 2;
Figure 4 shows a perspective view of the elastic connection of the trocar of Figure 2;
Figure 5 shows a perspective view from above of a first component of the body of the trocar of Figure 2;
Figure 6 shows a perspective view from below of a second component of the body of the trocar of Figure 2;
Figure 7 shows a perspective view from below of a third component of the body of the trocar of Figure 2;
Figure 8 shows a perspective view from below of the top cap of the trocar of Figure 2;
Figure 9 shows a perspective view from below of a fourth component of the body of the trocar of Figure 2;
Figure 10 shows a perspective view from above of a fifth component of the body of the trocar of Figure 2;
Figure 11 shows a perspective view from above of the seal device of the trocar of Figure 2;
Figure 12 shows a perspective exploded view of the seal device of Figure 11 and of the related housing shown in Figures 6 and 7;
Figure 13 shows a perspective view from above of first and second components of the seal device of Figure 11 (Fig. 13a) and a perspective view from below of the second component of the seal device of Figure 11 (Fig. 13b);
Figure 14 shows a perspective view of a third component of the seal device of Figure 11;
Figure 15 shows a perspective view from below of a fourth component of the seal device of Figure 11;
Figure 16 shows a top plan view of a fifth component of the seal device of Figure 11;
Figure 17 shows a top plan view (Fig. 17a), a rear view (Fig. 17b) and a section view along the cutting plane AA of Figure 17a of the third component of Figure 7;
Figure 18 shows a perspective view from above (Fig. 18a), a top plan view (Fig. 18b), a left side view (Fig. 18c), a right side view (Fig. 18d), and a rear view (Fig. 18e) of the fourth component of Figure 9; and
Figure 19 shows five perspective views of a portion of the trocar of Figure 2 in several configurations.

In the Figures, identical reference numbers are used for alike elements.

In particular, dimensions shown in the Figures are by way of example and have not to be intended as limiting the scope of protection of the present invention present invention, unless expressly indicated to the contrary.

In the following, explicit reference will be made to a preferred embodiment of the trocar according to the invention comprising a specific bladeless obturator with tip having helicoidal shape and a specifically shaped top cap. However, it is to be understood that the trocar according to the invention may comprise any other obturator, also provided with blades and with tip having a shape different from the described helicoidal one (e.g. a whistle shape), and a top cap shaped in any other way, still remaining within the scope of protection of the present invention.

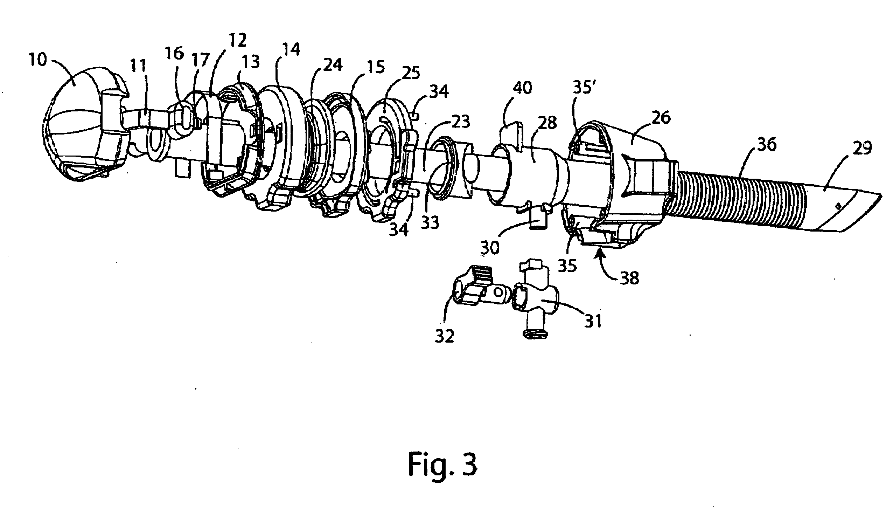

With reference to Figures 2 and 3, it may be observed that the preferred embodiment of the trocar according to the invention comprises a top cap 10 that may be coupled to a lower cap 13, the latter being centrally provided with a through hole, and to a first annular element 14 through an elastic connection 11 interacting with a U-shaped spring 12, as it will be described in detail in the following.

The first annular element 14 may be in turn coupled to a second annular element 15, so that the first and second annular elements 14 and 15 operate as container of a preferred embodiment of the seal device 24 according to the invention.

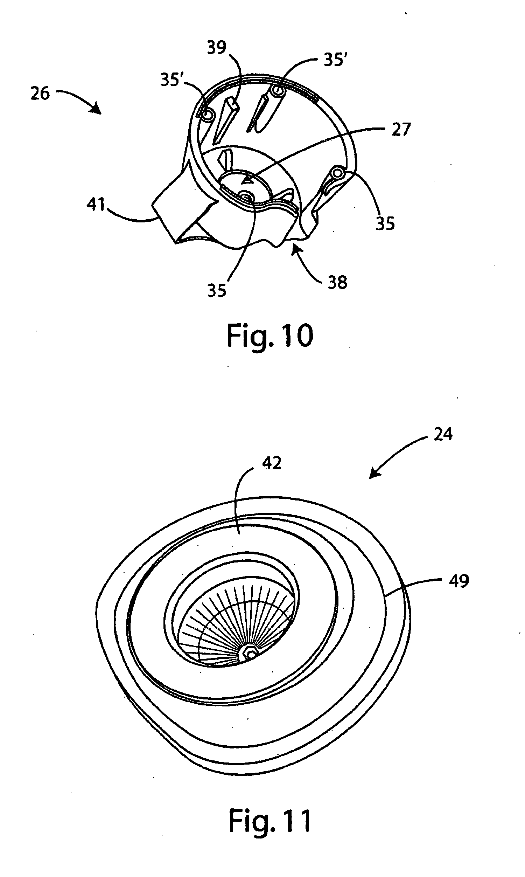

The trocar further comprises an annular cover 25 that is capable to close a container 26, provided at its bottom with a through hole (shown in Figure 10 and there indicated with the reference number 27), that houses the proximal end 28 of a hollow cylindrical cannula 29 (the distal end of which is flared, preferably by 45°). The proximal end 28 of the cannula 29 is provided with a front inlet/outlet duct 30 provided with a tap 31 controlled by a corresponding valve 32, for allowing the inside of the cannula 29 to connect, e.g., with external apparatuses of insufflation (e.g. for introducing gases). In this regard, a seal duckbill valve 33 is preferably housed in the proximal end 28 of the cannula 29. The outer wall of the cannula 29 is preferably centrally provided with a non slip machining constituted by rings 36 having a preferably triangular cross section. In particular, the assembly of components from the bottom cap 13 to the container 26 form the body of the trocar, indicated in Figure 1 with the reference number 5.

The obturator 23, ending with a distal tip 37 preferably having a specific helicoidal shape is capable to insert into the cannula 29.

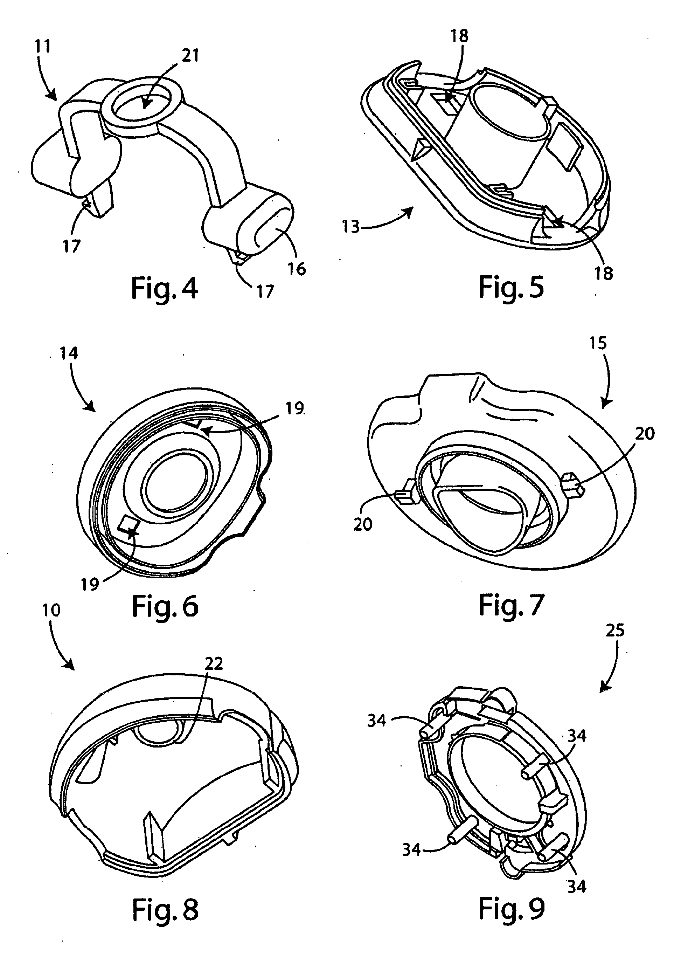

In particular, also with reference to Figure 4, it may be observed that the elastic connection 11 is U-shaped and has the two side arms which are provided, in proximity to the ends, with respective buttons 16 and which end with respective ridges 17 projecting outwards from the U. Making further reference to Figures 5 and 6, when the top cap 10 is assembled (as shown in Figure 2) with the lower cap 13 and the first annular' element 14, the end ridges 17 of the side arms of the elastic connection 11 are inserted in corresponding through holes 18 of the lower cap 13 and 19 of the first annular element 14; in this assembled configuration, when in rest position, the spring 12 keeps the side arms of the elastic connection 11 spaced apart (interacting with the surfaces of these facing the inside of the U), so that the end ridges 17 are locked in assembly position by the edges of the holes 19 of the first annular element 14. The top of the intermediate arm connected between the side arms of the elastic connection 11 is provided with a circular through hole 21 into which, in the assembled configuration, a corresponding circular projection 22 of the top of the inner surface of the top cap 10 (shown in Figure 8) is inserted, which constitutes the seat for fixing the trocar obturator 23. Starting from the assembled configuration of Figure 2, by pressing the two side buttons 16 so as to exceed the strength of the spring 12, it is possible to make the ridges 17 of the connection 11 disengage from the edges of the holes 19 of the first annular element 14 and of the holes 18 of the lower cap 13, making the side arms of the connection 11 slide and thus releasing the connection 11 and the top cap 10 from the trocar body, allowing the obturator 23 to be extracted.

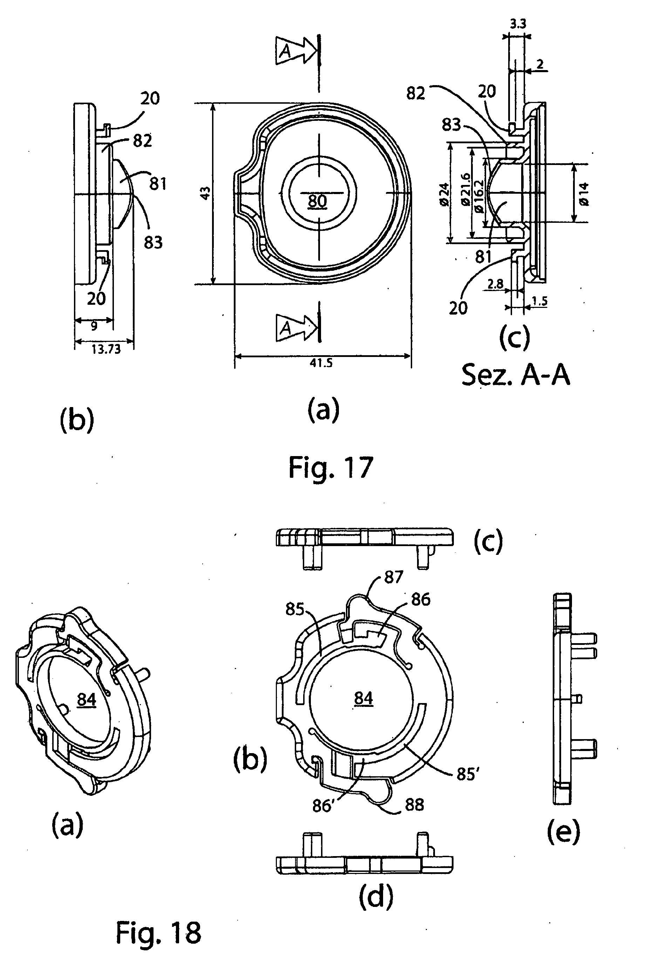

With reference to Figures 7 and 17, it may be observed that the second annular element 15 is provided, on the lower surface, with two ridges 20 and 20' arranged in diametrically opposed positions with respect to the central hole 80, around which, at radially increasing distance, a mechanical actuator 81 and a cylindrical relief 82 (less projecting than the actuator 81) are present. In particular, the mechanical actuator 81 is shaped according to a cylindrical surface sector the distal end of which is curvilinear so as to define two diametrically opposed curvilinear projections 83 (which are located, in the preferred embodiment of the trocar shown in the Figures, along a diameter orthogonal to that along which the two ridges 20 and 20' are located).

With reference to Figures 9 and 10 it may be observed that the annular cover 25 is provided at its bottom with four pins 34 and the container 26 is provided with four corresponding seats (the two front ones are indicated with 35 and the two rear ones with 35'): through the insertion of the pins 34 into the seats 35 and 35', the annular cover 25 is capable to be integrally coupled to the container 26. The container 26 is provided at its bottom with a through hole 27 (through which the cannula 29 may slide). The outer wall of the container 26 comprises a front notch 38, wherein the tap 31 and the respective valve 32 may be housed, thus determining the radial position of the cannula 29 within the container 26. Moreover, the inner wall of the container 26 comprises a rear rib 39 interacting with a rear tongue 40 of the proximal end 28 of the cannula 29, for determining, along with the cover 25, its longitudinal position within the container 26; in particular, the rear tongue 40 may further interact with the pair of rear seats 35' of the container 26 which (in combination with or alternatively to the housing of the duct 30-tap 31 assembly in the front notch 38) determine its radial position within the same container 26. As resulting also from Figure 2, the container 26 is provided at the distal end with two side projections 41 which allows the trocar to be better handled, as it will be described below.

With reference to Figure 18, it may be observed that the annular cover 25 is further provided, around the central hole 84, with two diametrically opposed guide grooves 85 and 85', shaped as a sector of annulus, each ending at an end with a constraint shaped area, respectively 86 and 86'. In correspondence with the constraint areas 86 and 86', the annular cover 25 comprises two side levers, respectively 87 and 88, which, when operated, allow to modify the shape of the respective areas 86 and 86', allowing the ridges 20 and 20' of the second annular element 25 to slide in the guide grooves 85 and 85' or to disengage the second annular element 25 from the cover 25, as it will be described in detail below.

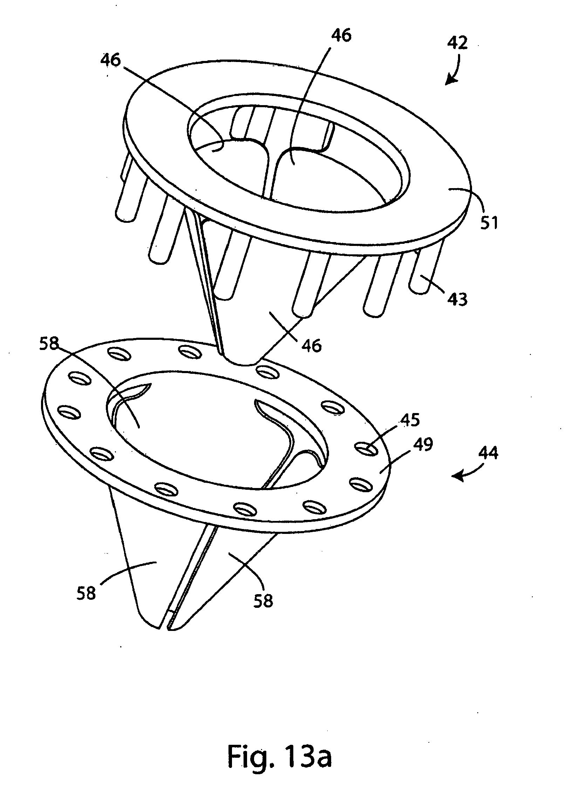

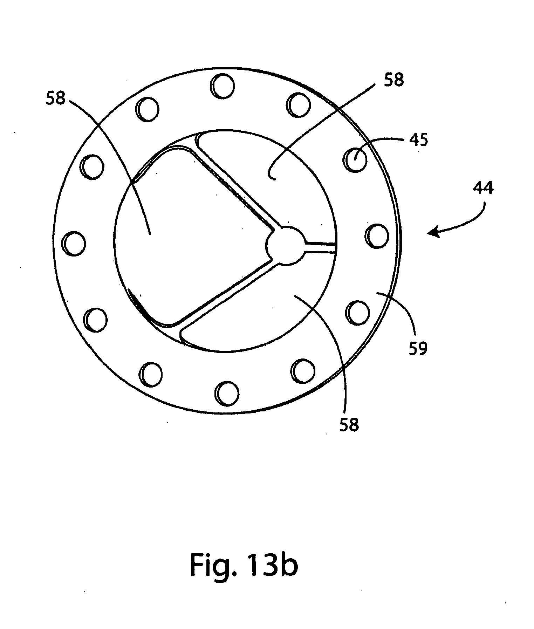

Figures 11-16 show the seal device 24 housed in the first and second annular elements 14 and 15. In particular, the seal device 24, shaped in accordance with the annular elements 14 and 15 (i.e. with circular plan slightly squeezed along a frontally placed circle arc, preferably ranging from 90° to 120°) is a self-centring seal device, comprising:
- a proximal truncated conical element 42, of rigid elastic material, preferably in thermoplastic elastomer such as Hytrel available from DuPont® and/or polycarbonate, composed by three conical sectors 46 connected to a proximal circular rim 51 provided on the distal surface with a plurality of (preferably twelve) pins 43;
- a proximal membrane 49 (with a plan shaped in accordance with the annular elements 14 and 15), of soft elastic material, preferably in silicone and/or laprene and/or in a mix of styrene butadiene rubber (SBR) and nitrile rubber (NBR) (having more preferably hardness equal to 40 shore A), centrally provided with a through hole 62 delimited by a circular rim 60 provided with a plurality of (preferably twelve) through holes 50, from which a projecting edge 61 (orientated towards the distal end of the trocar) extends;
- a distal truncated conical element 44, of rigid elastic material, preferably equal to the one of the proximal truncated conical element 42, more preferably in thermoplastic elastomer such as Hytrel available from DuPont® and/or polycarbonate, composed by three conical sectors 58 connected to a proximal circular rim 59 provided with a plurality of (preferably twelve) through holes 45; in particular, the through holes 45 are aligned with the pins 43 of the proximal truncated conical element 42 so as to allow the two proximal and distal truncated conical elements 42 and 44 to overlap the respective three conical sectors 46 and 58 rotated from each other by 60°;
- a distal membrane with three truncated conical elements 55, of soft elastic material, preferably equal to the one of the proximal membrane 49, more preferably in silicone and/or laprene and/or in a mix of styrene butadiene rubber (SBR) and nitrile rubber (NBR) (still more preferably having hardness equal to 40 shore A), overlapping each other, wherein each truncated conical element 55 comprises a conical surface 52 provided with a linear slit 56 aligned with a generating line of the same conical surface, the three truncated conical elements 55 being overlapping each other with the respective slits rotated from each other by 120°, the conical surface 52 of each truncated conical element 55 being connected to a proximal circular rim 53, provided with a plurality of (preferably twelve) through holes 54, the distal end of the conical surface 52 of each truncated conical element 55 being joined to a cylindrical surface 57;
- a distal annular bracket 47, of rigid plastic material, preferably in thermoplastic polymer, more preferably in acrylonitrile butadiene styrene or ABS and/or polycarbonate, provided with a plurality of (preferably twelve) through holes 48.

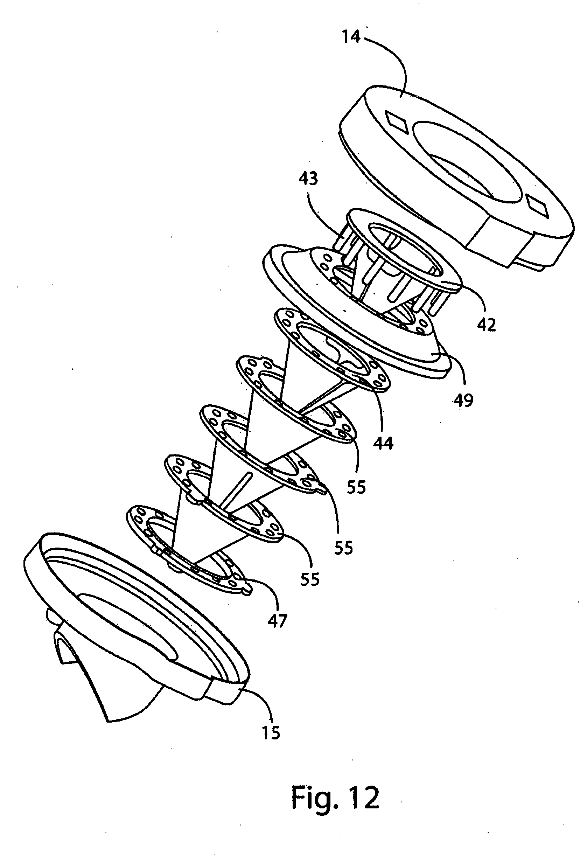

As better shown in Figure 12, the proximal membrane 49 is located between the proximal truncated conical element 42 and the distal truncated conical element 44, the proximal circular rim 59 of the distal truncated conical element 44 being housed in the proximal membrane 49 in contact with the distal surface of the circular rim 60 and internally to the projecting edge 61 of the latter. The distal membrane with three truncated conical elements 55 is located between the distal truncated conical element 44 and the distal bracket 47. The pins 43 of the proximal truncated conical element 42 insert into the holes 50 of the proximal membrane 49, into the holes 45 of the distal truncated conical element 44, into the holes 54 of the three truncated conical elements 55 of the distal membrane, and into the holes 48 of the distal bracket 47; preferably, the pins 43 are pressed into the aforementioned holes and swaged.

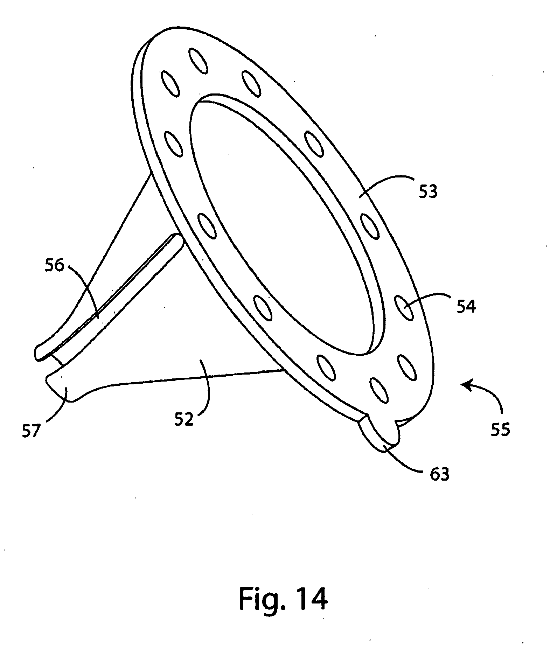

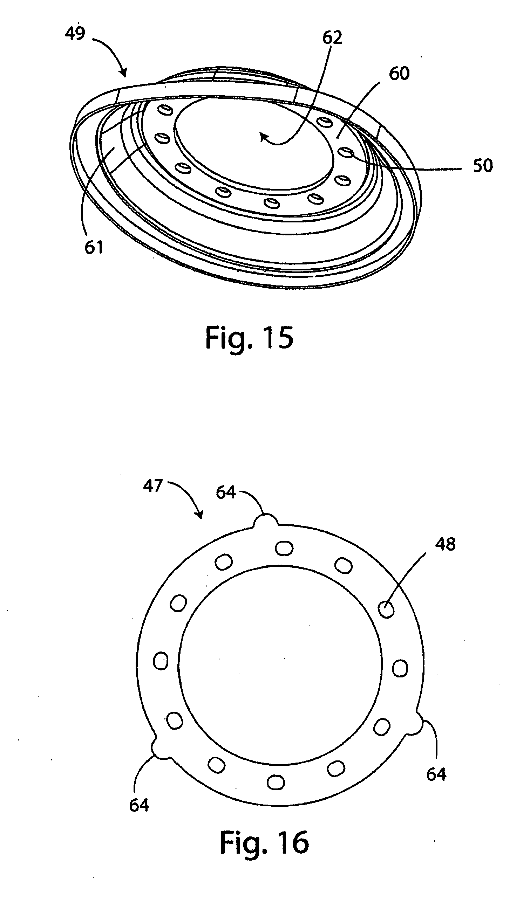

The linear slits 56 of the three truncated conical elements 55 of the distal membrane, as said rotated from each other by 120°, allow the expansion of the three conical surfaces 52 forming the distal membrane for adapting to the different diameters of the surgical instruments inserted into the trocar. In particular, the proximal circular rim 53 of each one of the three truncated conical elements 55 is provided with a peripheral projection 63 (see Figure 14), and the distal bracket 47 is provided with three corresponding peripheral projections 64 (see Figure 16) angularly spaced from each other by 120°: the peripheral projections 64 aid in correctly overlapping the three truncated conical elements 55 (i.e., with the slits rotated from each other by 120°), since it is sufficient to align each one of the peripheral projections 63 of the latter with a respective peripheral projection 64 of the distal bracket 47.

The two proximal and distal truncated conical elements 42 and 44 (as said, mounted one inside the other with the respective three conical sectors 46 and 58 rotated from each other by 60°), are inserted within the three truncated conical elements 55 of the distal membrane for preventing the same three truncated conical elements 55 of the distal membrane from rolling up along proximal direction when a surgical instrument previously inserted into the trocar is then extracted from the latter.

The proximal membrane 49 located between the two proximal and distal truncated conical elements 42 and 44 ensures the hydraulic tightness of the device 24 with respect to the housing formed by the annular elements 14 and 15. Also, the proximal membrane 49 is radially mobile within the housing formed by the annular elements 14 and 15, allowing the whole device 24 when assembled to be identically mobile. This allows the surgical instruments to be inserted within the patient's abdominal cavity through the trocar with various angle with respect to the longitudinal axis of the cannula 29. Preferably, the annular elements 14 and 15, more preferably in polycarbonate, are stuck to each other.

In particular, when the obturator 13 is inserted into the cannula 29 or at least one surgical instrument is inserted within the patient's abdominal cavity through the trocar, it is the device 24 that ensures the sealing of the abdominal cavity (avoiding leakage of biological fluids, usually maintaining the pressure difference with the exterior). Instead, when the obturator 13 is not inserted into the cannula 29 nor any other surgical instrument is inserted within the abdominal cavity, it is the duckbill valve 33 that ensures the sealing of the abdominal cavity.

Other embodiments of the seal device 24 according to the invention may have the proximal truncated conical element 42 and the distal truncated conical element 44 which have a different number of conical sectors from each other, and which preferably have, when mounted one inside the other, the respective conical sectors rotated from each other so that the separating spaces between the respective conical sectors are offset (i.e. not aligned).

Other embodiments of the seal device 24 according to the invention may have the proximal truncated conical element 42 and the distal truncated conical element 44 which have one or more conical sectors, in number different from three. If the proximal truncated conical element 42 and the distal truncated conical element 44 have the same number of conical sectors, these are preferably rotated from each other so that the separating spaces between the respective conical sectors are offset (i.e. not aligned), and they are more preferably rotated from each other by an angle equal to the half of the ratio between 360° and the number of conical sectors of each one of the proximal and distal truncated conical elements 42 and 44. If the proximal truncated conical element 42 and the distal truncated conical element 44 have a different number of conical sectors from each other, when mounted one inside the other they preferably have the respective conical sectors rotated from each other so that the separating spaces between the respective conical sectors are offset (i.e. not aligned).

Further embodiments of the seal device 24 according to the invention may have the distal membrane that has a number of overlapping truncated conical elements that is larger than one and different from three. In this case, the truncated conical elements are overlapping each other with the respective slits rotated from each other by an angle equal to the ratio between 360° and the number of overlapping truncated conical elements.

Other embodiments of the seal device 24 according to the invention may have each one of the overlapping truncated conical elements that has two or more linear slits aligned along different directrix lines, preferably angularly equally spaced from each other. Further embodiments of the seal device 24 according to the invention may also have at least one of the truncated conical elements that has no linear slit, most of all in the case where the material of such truncated conical element has specific elastic properties.

Still with reference to Figure 2, it may be observed that the distal tip 37 of the obturator 23 advantageously has a specific helicoidal shape and the vertex 70 of the tip 37 is misaligned with respect to the longitudinal axis of the obturator 23. The top cap 10 has a shape that makes the handle of the trocar extremely handy and ergonomic. The whole height of the handle of the trocar according to the invention may be easily adjusted by varying the heights of the component elements, from the top cap 10 down to the container 26, in order to improve handiness and ergonomics of the handle of the trocar.

With regard to the materials with which the trocar components are made, the top and lower caps 10 and 13, the connection 11, the first and second annular elements 14 and 15, the obturator 23, the cover 25, the container 26, the cannula 29, and the tap 31 are preferably made of polycarbonate; the spring 12 is preferably made of spring steel (i.e. still with high rate of carbon, preferably ranging from 0,80% to 0,90 %, hence particularly hard); the valve 32 of the tap 31 is preferably made of polyethylene; the duckbill valve 33 is preferably made of silicone. Moreover, the trocar is assembled by using adhesives of biocompatible type.

In general, the device is mostly made by using plastic materials obtained through molding, and the parts are then assembled through glueing, through fixing by pressing or through using screws.

The modes of use of the trocar according to the invention are extremely simple. In fact, still making reference to Figure 2, the surgeon handles the trocar by placing the rear part of the top cap 10 and of the body within the hand palm and using the two distal side projections 41 for having a grip with the fingers (preferably middle and ring fingers); in other words, the trocar body is closed within the fist whereas the cannula 29 thereof comes out from the space between two fingers pressing the two distal side projections 41. During the phase of pushing the trocar, it is advantageous to also impart a slight torsional clockwise movement, facilitating the penetration of the tip 37 of the obturator 23 and of the cannula 29 into the abdominal cavity, the tissues of the walls of which are spaced apart without lacerations thanks to the specific helicoidal shape of the tip 37. This torsional movement further allows the central helicoidal thread 36 of the cannula 29 to adhere to the abdominal walls, facilitating the mechanical coupling of the trocar to the abdomen and eliminating the need for suture stitches or clips for fixing the cannula 29 to the abdominal wall.

Once the introduction of the trocar according to the invention into the abdominal cavity has been completed, the surgeon presses (e.g. with thumb and index finger of a hand) the two side buttons 16 of the connection 11 thus removing the top cap 10 and extracting the obturator 23. During the extraction of the obturator 23, leakage of biological fluids is avoided by the duckbill valve 33 and the seal device 24.

Then, when the obturator 23 has been extracted, the cannula 29 becomes channel for allowing passage of several surgical instruments. It is also possible to introduce medical gases through the tap 31, by connecting it with external insufflation apparatuses preferably through standard *luer lock* connection.

As said, the use of the trocar according to the invention is facilitated by the shape of the same, that allows a handy and ergonomic handling, wherein the top cap 10 is capable to be stably housed within the hand palm and the two distal side projections 41 offer firm leverage points for the fingers (preferably middle and ring fingers) so as to allow a reliable control by both left- and right-handed surgeons.

With reference to Figures 7, 9 and 17-19, it may be observed that the second annular element 15 (coupled to the first annular element 14 so as to contain the seal device 24) and the annular cover 25, in correspondence of which the duckbill valve 33 is, are rotatably coupled to each other so that, thanks to the lower shape of the second annular element 15, they may assume a configuration wherein the duckbill valve 33, that in its rest position is closed, is open.

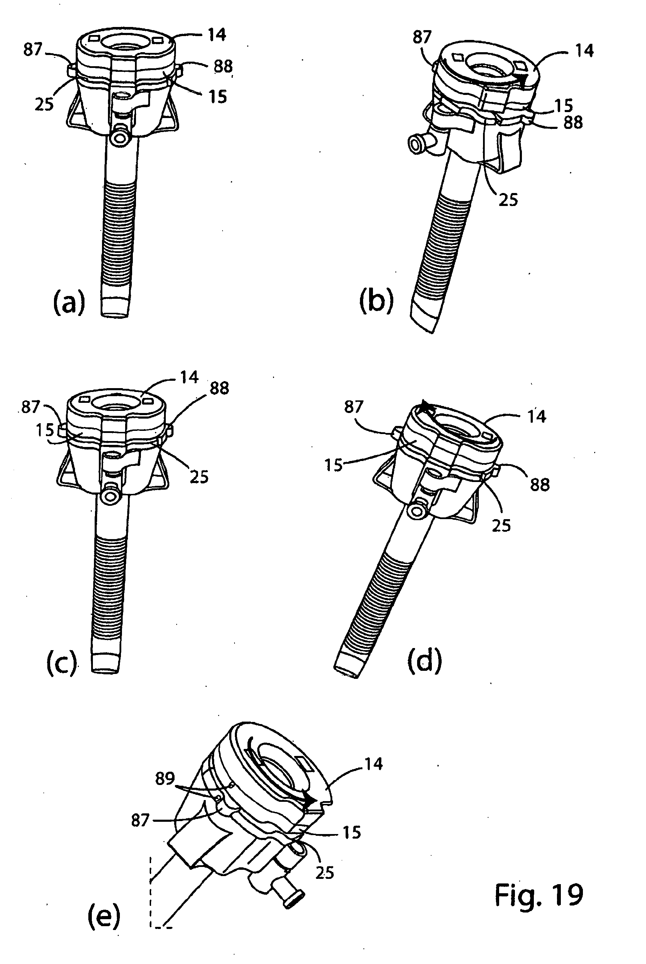

In the assembled configuration of Figure 2, wherein, as shown in Figure 19a, the second annular element 15 (and the first annular element 14) and the annular cover 25 are aligned with each other, the two ridges 20 and 20' of the former are locked in the constraint areas 86 and 86' of the latter.

Starting from this assembled configuration, by keeping the left side opening lever 87 pressed, the shape of the constraint area 86 of the cover 25 is modified allowing the left ridge 20 of the second annular element 15 to disengage from such area 86 and allowing the ridges 20 and 20' of the second annular element 15 to slide within the grooves 85 and 85' of the cover 25, thus rotating the second annular element 15 (and the first annular element 14) anticlockwise with respect to the cover 25 as shown by the arrow in Figure 19b. In this configuration, the two curvilinear projections 83 of the mechanical actuator 81 of the second annular element 15 space the lips of the valve 33 apart thus opening it. From this configuration, it is sufficient to rotate the second annular element 15 (and the first annular element 14) clockwise with respect to the cover 25 for returning to the assembled configuration of Figures 2 and 19a.

In order to remove the first and second annular elements 14 and 15, and the seal device 24 contained therein, it is sufficient, starting from the assembled configuration shown in Figure 19c, keeping the right side closing lever 88 pressed, in such a way that the shape of the constraint area 86' of the cover 25 is modified allowing the right ridge 20' of the second annular element 15 to disengage from such area 86' and allowing the ridges 20 and 20' of the second annular element 15 to slightly rotate clockwise with respect to the cover 25 as shown by the arrow in Figure 19d. In this configuration, the two curvilinear projections 83 of the mechanical actuator 81 of the second annular element 15 space the lips of the valve 33 apart thus opening it. In this configuration, both ridges 20 and 20' are disengaged and free to slide upwards, allowing the second annular element 15 (along with the first annular element 14 and the seal device 24 contained therein) to be uncoupled from the cover 25.

In order to insert the second annular element 15 (along with the first annular element 14 and the seal device 24 contained therein) on the cover 25, it is sufficient to insert the ridges 20 and 20' of the second annular element 15 in the constraint areas 86 and 86' of the cover 25, keeping the second annular element 15 and the cover in the same angular orientation that they have when they are separated (as shown in Figure 19d), and subsequently to rotate the second annular element 15 (along with the first annular element 14 and the seal device 24 contained therein) anticlockwise with respect to the cover 25 as shown by the arrow of Figure 19e, until the assembled configuration (shown in Figure 19a), wherein they are aligned, is reached. Advantageously, two indicators (e.g. two arrows) 89 on the side surface of the second annular element 15 and of the cover 25 may indicate the correct angular orientation of these.

The preferred embodiments have been above described and some modifications of this invention have been suggested, but it should be understood that those skilled in the art can make variations and changes, without so departing from the related scope of protection, as defined by the following claims.

## Claims

1. Seal device (24) for trocar, comprising, in the following order:
- a proximal truncated conical element (42), of rigid elastic material, composed of a plurality of conical sectors (46),
- a distal truncated conical element (44), of rigid elastic material, composed of a plurality of conical sectors (58),
- a distal membrane with two or more truncated conical elements (55), of soft elastic material, overlapping each other, each one of which comprises a conical surface (52) and
- a distal annular bracket (47), of rigid plastic material,
wherein
the device (24) further comprises a proximal annular-shaped membrane (49), of soft elastic material, centrally provided with a through hole (62) and coupling mechanical means (43, 45, 48, 50, 51, 53, 54, 59, 60, 61) coupling the proximal truncated conical element (42), the proximal membrane (49), the distal truncated conical element (44), said two or more truncated conical elements (55) of said distal membrane and the distal annular bracket (47) to each other, whereby the proximal truncated conical element (42) is inserted in the distal truncated conical element (44) that is in turn inserted in said distal membrane,
wherein each one of said truncated conical elements (55) is provided with a linear slit (56) aligned with a generating line of its conical surface (52), said two or more truncated conical elements (55) being overlapped one another with the respective linear slits (56) rotated from each other so as to be misaligned.
and wherein the proximal truncated conical element (42) is inserted in the distal truncated conical element (44) so that their respective conical sectors (46, 58) are rotated from each other so that separating spaces between the conical sectors (46) of the proximal truncated conical element (42) are misaligned with respect to the conical sectors (58) of the proximal truncated conical element (44).

2. Seal device (24) according to the preceding claim, **characterised in that** the linear slits (56) of said two or more truncated conical elements (55) are rotated from each other by an angle equal to the ratio between 360° and the number of said two or more truncated conical elements (55).

3. Seal device (24) according to claim 1 or 2, **characterised in that** said two or more truncated conical elements (55) of said distal membrane are three in number.

4. Seal device (24) according to any one of the preceding claims, **characterised in that** the proximal truncated conical element (42) is composed of at least two, preferably three, conical sectors (46) and **in that** the distal truncated conical element (44) is composed of at least two, preferably three, conical sectors (58), the proximal truncated conical element (42) and the distal truncated conical element (44) being more preferably composed of a same number of respective conical sectors, rotated from each other by an angle still more preferably equal to the half of the ratio between 360° and said same number of conical sectors.

5. Seal device (24) according to any one of the preceding claims, **characterised in that** said coupling mechanical means (43, 45, 48, 50, 51, 53, 54, 59, 60, 61) comprises at least one plurality of pins (43) inserted in one or more corresponding pluralities of holes (45, 48, 50, 54), said pins (43) being preferably pressed in said holes (45, 48, 50, 54) and swaged, more preferably:
- the proximal truncated conical element (42) comprising a proximal circular rim (51), to which said one or more conical sectors (46) are connected, provided on a distal surface with a plurality of pins (43),
- the proximal membrane (49) comprising a circular rim (60), delimiting the central through hole (62), provided with a plurality of through holes (50) corresponding to said plurality of pins (43),
- the distal truncated conical element (44) comprising a proximal circular rim (59), to which said one or more conical sectors (58) are connected, provided with a plurality of through holes (45) corresponding to said plurality of pins (43),
- each one of said two or more truncated conical elements (55) comprising a proximal circular rim (53), to which the conical surface (52) is connected, provided with a plurality of through holes (54) corresponding to said plurality of pins (43),
- the distal annular bracket (47) being provided with a plurality of holes (48), still more preferably through holes, corresponding to said plurality of pins (43).

6. Seal device (24) according to any one of the preceding claims, **characterised in that** the proximal membrane (49) comprises a circular rim (60), delimiting the central through hole (62), from which a projecting edge (61) extends, a proximal end (59) of the distal truncated conical element (44) being housed in the proximal membrane (49) in contact with a distal surface of the circular rim (60) thereof and internally to the projecting edge (61) of the latter, the proximal membrane (49) having a shape with circular plan that is frontally slightly squeezed along a circle arc, preferably ranging from 90° to 120°.

7. Seal device (24) according to any one of the preceding claims, **characterised in that** a distal end of the conical surface (52) of at least one of said two or more truncated conical elements (55) of said distal membrane is linked to a cylindrical surface (57).

8. Seal device (24) according to any one of the preceding claims, **characterised in that** it is housed in a first and a second annular elements (14, 15), coupled to each other to form a container of the device (24) and having a central through hole, the first and second annular elements (14, 15) being preferably of polycarbonate, the first and second annular elements (14, 15) being more preferably glued to each other.

9. Seal device (24) according to any one of the preceding claims, **characterised in that**:
- the proximal truncated conical element (42) is made of at least one material selected from the group comprising a thermoplastic elastomer, preferably Hytrel from DuPont(R), and polycarbonate,
and/or
- the proximal membrane (49) is made of at least one material selected from the group comprising silicone, laprene, mix of styrene butadiene rubber (SBR) and nitrile rubber (NBR), the material of the proximal membrane (49) having preferably hardness equal to 40 shore A, and/or
- the distal truncated conical element (44) is made of thermoplastic elastomer, preferably Hytrel from DuPont(R), and/or polycarbonate, and/or
- said two or more truncated conical elements (55) of said distal membrane are made of at least one material selected from the group comprising silicone, laprene, mix of styrene butadiene rubber (SBR) and nitrile rubber (NBR), the material of the proximal membrane (49) having preferably hardness equal to 40 shore A, and/or
- the distal annular bracket (47) is made of at least one material selected from the group comprising a thermoplastic polymer, preferably acrylonitrile butadiene styrene or ABS, and polycarbonate.

10. Trocar comprising a body (13, 14, 15, 25, 26), coupled to a hollow cylindrical cannula (29) in which an obturator (23) is insertable, and a top cap (10), having a top, that is removably coupled to the body (13, 14, 15), **characterised in that** the body (13, 14, 15, 25, 26) comprises a seal device (24) according to any one of claims 1 to 9.

## Patentansprüche

1. Dichtungsvorrichtung (24) für einen Trokar, umfassend, in der folgenden Reihenfolge:
- ein proximales, kegelstumpfförmiges Element (42) aus einem festen, elastischen Material, zusammengesetzt aus einer Mehrzahl von konischen Sektoren (46),
- ein distales, kegelstumpfförmiges Element (44) aus einem festen, elastischen Material, zusammengesetzt aus einer Mehrzahl von konischen Sektoren (58),
- eine distale Membran mit zwei oder mehr, einander überlappenden, kegelstumpfförmigen Elementen (55) aus einem weichen, elastischen Material, von denen jedes eine konische Oberfläche (52) aufweist, und
- eine distale, ringförmige Klammer (47) aus einem festen Kunststoffmaterial,
wobei
die Vorrichtung (24) ferner eine proximale, ringförmige Membran (49) aus einem weichen, elastischen Material aufweist, die im Zentrum mit einem Durchgangsloch (62) versehen ist sowie mit einem mechanischen Koppelmittel (43, 45, 48, 50, 51, 53, 54, 59, 60), welches das proximale, kegelstumpfförmige Element (42), die proximale Membran (49), das distale, kegelstumpfförmige Element (44), die besagten zwei oder mehr kegelstumpfförmigen Elemente (55) der besagten, distalen Membran und die distale, ringförmige Klammer (47) miteinander koppelt, wobei das proximale, kegelstumpfförmige Element (42) eingesetzt wird in das distale, kegelstumpfförmige Element (44), welches seinerseits in die besagte, distale Membran eingesetzt wird,
wobei jedes der besagten kegelstumpfförmigen Elemente (55) mit einem linearen Schlitz (56) versehen ist in einer Flucht mit einer erzeugenden Linie seiner konischen Oberfläche (52), wobei die besagten zwei oder mehr kegelstumpfförmigen Elemente (55) einander derart überlappen, dass die jeweiligen, linearen Schlitze (56) gegeneinander verdreht sind und dadurch nicht miteinander fluchten,
und wobei das proximale, kegelstumpfförmige Element (42) in das distale, kegelstumpfförmige Element (44) derart eingesetzt ist, dass ihre jeweiligen konischen Sektoren (46, 58) voneinander weggedreht sind, so dass trennende Abstände zwischen den konischen Sektoren (46) des proximalen, kegelstumpfförmigen Elements (42) gegenüber den konischen Sektoren (58) des proximalen, kegelstumpfförmigen Elements (44) verstellt sind.

2. Dichtungsvorrichtung (24) gemäß dem vorangehenden Anspruch, **dadurch gekennzeichnet, dass** die linearen Schlitze (56) der besagten zwei oder mehr kegelstumpfförmigen Elemente (55) um einen Winkel gleich dem Verhältnis zwischen 360° und der Anzahl der besagten zwei oder mehr kegelstumpfförmigen Elemente (55) voneinander weggedreht sind.

3. Dichtungsvorrichtung (24) gemäß Anspruch 1 oder 2, **dadurch gekennzeichnet, dass** die besagten zwei oder mehr kegelstumpfförmigen Elemente (55) der besagten distalen Membran drei in der Anzahl sind.

4. Dichtungsvorrichtung (24) gemäß einem der vorangehenden Ansprüche, **dadurch gekennzeichnet, dass** das proximale kegelstumpfförmige Element (42) aus wenigstens zwei, vorzugsweise drei, konischen Sektoren (46) zusammengesetzt ist, sowie dadurch, dass das distale kegelstumpfförmige Element (44) aus wenigstens zwei, vorzugsweise drei, konischen Sektoren (58) zusammengesetzt ist, wobei das proximale kegelsumpfförmige Element (42) und das distale kegelstumpfförmige Element (44) besonders bevorzugt aus einer gleichen Anzahl von jeweiligen konischen Sektoren bestehen, welche gegeneinander um einen Winkel verdreht sind, der ganz besonders bevorzugt gleich der Hälfte des Verhältnisses zwischen 360° und der besagten Anzahl von konischen Sektoren ist.

5. Dichtungsvorrichtung (24) gemäß einem der vorangehenden Ansprüche, **dadurch gekennzeichnet, dass** das besagte mechanische Koppelmittel (43, 45, 48, 50, 51, 53, 54, 59, 60, 61) wenigstens eine Mehrzahl von in eine oder mehrere entsprechende Mehrzahlen von Öffnungen (45, 48, 50, 54) eingesetzten Stiften (43) umfasst, wobei die besagten Stifte (43) vorzugsweise in die besagten Öffnungen (45, 48, 50, 54) eingepresst und gestaucht sind, im Besonderen:
- wobei das proximale kegelstumpfförmige Element (42) einen proximalen kreisförmigen Rand (51) aufweist, womit besagte ein oder mehrere Sektoren (46) verbunden sind, welche auf einer distalen Oberfläche mit einer Mehrzahl von Stiften (43) vorgesehen sind,
- wobei die proximale Membran (49) einen kreisförmigen Rand (60) aufweist, der die zentrale Durchgangsöffnung (62) abgrenzt, und mit einer Mehrzahl von Durchgangsöffnungen (50) versehen ist, die mit der besagten Mehrzahl von Stiften (43) korrespondieren,
- wobei das distale kegelstumpfförmige Element (44) einen proximalen kreisförmigen Rand (59) aufweist, womit besagte ein oder mehrere Sektoren (58) verbunden sind, welche mit einer Mehrzahl von Durchgangsöffnungen (45) entsprechend der besagten Mehrzahl von Stiften (43) versehen sind,
- wobei jedes der besagten, zwei oder mehr kegelstumpfförmigen Elemente (55) mit proximalem, kreisförmigen Rand (53), womit die konische Oberfläche (52) verbunden ist, mit einer Mehrzahl von Durchgangsöffnungen (54) entsprechend der besagten Mehrzahl von Stiften (43) versehen ist,
- und wobei die distale, ringförmige Klammer (47) mit einer Mehrzahl von Öffnungen (48) versehen ist, bevorzugt mit Durchgangsöffnungen, entsprechend der Mehrzahl von Stiften (43).

6. Dichtungsvorrichtung (24) gemäß einem der vorangehenden Ansprüche, **dadurch gekennzeichnet, dass** die proximale Membran (49) einen kreisförmigen Rand (60) aufweist, welcher die zentrale Durchgangsöffnung (62) begrenzt, wovon sich eine hervorstehende Kante (61) erstreckt, wobei ein proximales Ende (59) des distalen, kegelstumpfförmigen Elements (44) in der proximalen Membran (49) aufgenommen ist in Kontakt mit einer distalen Oberfläche von deren kreisförmigen Randes (60), sowie innerhalb einer vorspringenden kante (61) des letzteren, und wobei die proximale Membran (49) eine Gestalt mit einer kreisförmigen Ebene hat, die an der Vorderseite entlang eines Kreisbogens leicht gestaucht ist, vorzugsweise in einem Bereich von 90° bis 120°.

7. Dichtungsvorrichtung (24) gemäß einem der vorangehenden Ansprüche, **dadurch gekennzeichnet, dass** das distale Ende der konischen Oberfläche (52) wenigstens eines der besagten zwei oder mehr kegelstumpfförmigen Elemente (55) der besagten distalen Membran mit einer zylindrischen Oberfläche (57) verbunden ist.

8. Dichtungsvorrichtung (24) gemäß einem der vorangehenden Ansprüche, **dadurch gekennzeichnet, dass** sie umhaust ist von ersten und zweiten, ringförmigen Elementen (14, 15), welche miteinander gekoppelt sind, um einen Behälter der Vorrichtung (24) zu bilden, und welche eine zentrale Durchgangsöffnung aufweisen, wobei die ersten und zweiten ringförmigen Elemente (14, 15) bevorzugt aus Polykarbonat bestehen, und wobei die ersten und zweiten ringförmigen Elemente (14, 15) insbesondere zusammengeklebt sind.

9. Dichtungsvorrichtung (24) gemäß einem der vorangehenden Ansprüche, **dadurch gekennzeichnet, dass**:
- das proximale, kegelstumpfförmige Element (42) aus wenigstens einem Material hergestellt ist, ausgewählt aus der Gruppe umfassend ein thermoplastisches Elastomer, vorzugsweise Hytrel von DuPont®, und Polykarbonat, und/oder
- die proximale Membran (49) aus wenigstens einem Material hergestellt ist, ausgewählt aus der Gruppe umfassend Silikon, Lapren, einer Mischung von Stryrol-Butadien-Gummi (SBR) und Nitrilkautschuk (NBR), wobei das Material der proximalen Membran (49) vorzugsweise eine Shore-Härte gleich 40 A hat, und/oder
- das distale, kegelstumpfförmige Element (44) aus wenigstens einem Material hergestellt ist, ausgewählt aus der Gruppe umfassend ein thermoplastisches Elastomer, vorzugsweise Hytrel von DuPont®, und Polykarbonat, und/oder
- die besagten, zwei oder mehr kegelstumpfförmigen Elemente (55) der besagten distalen Membran aus wenigstens einem Material hergestellt sind, ausgewählt aus der Gruppe umfassend Silikon, Lapren, einer Mischung von Stryrol-Butadien-Gummi (SBR) und Nitrilkautschuk (NBR), wobei das Material der proximalen Membran (49) vorzugsweise eine Shore-Härte gleich 40 A hat, und/oder
- die distale, ringförmige Klammer (47) aus wenigstens einem Material hergestellt ist, ausgewählt aus der Gruppe umfassend ein thermoplastisches Elastomer, vorzugsweise Acrylnitril-Butadien-Styrol oder ABS, und Polykarbonat.

10. Trokar, umfassend einen Körper (13, 14, 15, 25, 26), angekoppelt an eine hohle zylindrische Kanüle (29), worin ein Obturator (23) einsetzbar ist, und eine obere Kappe (10) mit einer Oberseite, die abnehmbar mit einem Körper (13, 14, 15) gekoppelt ist, **dadurch gekennzeichnet, dass** der Körper (13, 14, 15, 25, 26) eine Dichtungsvorrichtung (24) gemäß einem der Ansprüche 1 bis 9 umfasst.

## Revendications

1. Dispositif d'étanchéité (24) pour trocart, comprenant, dans l'ordre suivant :
un élément tronconique proximal (42), en matériau élastique rigide, composé d'une pluralité de secteurs coniques (46),
un élément tronconique distal (44) en matériau élastique rigide, composé d'une pluralité de secteurs coniques (58),
une membrane distale avec deux éléments tronconiques (55) ou plus en matériau élastique souple, se chevauchant, dont chacun comprend une surface conique (52), et
un support annulaire distal (47) en matière plastique rigide, dans lequel :
le dispositif (24) comprend en outre une membrane de forme annulaire proximale (49) en matériau élastique souple, prévue de manière centrale avec un trou débouchant (62) et des moyens mécaniques de couplage (43, 45, 48, 50, 51, 53, 54, 59, 60, 61) couplant l'élément tronconique proximal (42), la membrane proximale (49), l'élément tronconique distal (44), lesdits deux éléments tronconiques (55) ou plus de ladite membrane distale et le support annulaire distal (47) entre eux, moyennant quoi l'élément tronconique proximal (42) est inséré dans l'élément tronconique distal (44), qui est à son tour inséré dans ladite membrane distale,
dans lequel chacun desdits éléments tronconiques (55) est prévu avec une fente linéaire (56) alignée avec une ligne génératrice de sa surface conique (52), lesdits deux éléments tronconiques (55) ou plus se chevauchant entre eux, avec les fentes linéaires (56) respectives tournées les unes par rapport aux autres afin de présenter un défaut d'alignement,
et dans lequel l'élément tronconique proximal (42) est inséré dans l'élément tronconique distal (44) de sorte que leurs secteurs coniques (46, 58) respectifs sont tournés les uns par rapport aux autres de sorte que des espaces de séparation entre les secteurs coniques (46) de l'élément tronconique proximal (42) présentent un défaut d'alignement par rapport aux secteurs coniques (58) de l'élément tronconique proximal (44).

2. Dispositif d'étanchéité (24) selon la revendication précédente, **caractérisé en ce que** les fentes linéaires (56) desdits deux éléments tronconiques (55) ou plus sont tournées les unes par rapport aux autres selon un angle égal au rapport compris entre 360° et le nombre desdits deux éléments tronconiques (55) ou plus.

3. Dispositif d'étanchéité (24) selon la revendication 1 ou 2, **caractérisé en ce que** lesdits deux éléments tronconiques (55) ou plus de ladite membrane distale sont au nombre de trois.

4. Dispositif d'étanchéité (24) selon l'une quelconque des revendications précédentes, **caractérisé en ce que** l'élément tronconique proximal (42) est composé d'au moins deux secteurs tronconiques (46) de préférence trois, et ce que ledit élément tronconique distal (44) est composé d'au moins deux, de préférence trois, secteurs coniques (58), l'élément tronconique proximal (42) et l'élément tronconique distal (44) étant encore de préférence composés du même nombre de secteurs coniques respectifs, tournés l'un par rapport à l'autre selon un angle encore de préférence égal à la moitié du rapport compris entre 360° et ledit même nombre de secteurs coniques.

5. Dispositif d'étanchéité (24) selon l'une quelconque des revendications précédentes, **caractérisé en ce que** ledit moyen mécanique de couplage (43, 45, 48, 50, 51, 53, 54, 59, 60, 61) comprend au moins une pluralité de broches (43) insérées dans une ou plusieurs pluralités correspondantes de trous (45, 48, 50, 54), lesdites broches (43) étant de préférence comprimées dans lesdits trous (45, 48, 50, 54) et embouties, plus préférablement :
- l'élément tronconique proximal (42) comprenant un rebord circulaire proximal (51), auquel lesdits uns ou plusieurs secteurs coniques (46) sont raccordés, prévu sur une surface distale avec une pluralité de broches (43),
- la membrane proximale (49) comprenant un rebord circulaire (60), délimitant le trou débouchant central (62), prévue avec une pluralité de trous débouchants (50) correspondant à ladite pluralité de broches (43),
- l'élément tronconique distal (44) comprenant un rebord circulaire proximal (59) auquel lesdits uns ou plusieurs secteurs coniques (58) sont raccordés, prévu avec une pluralité de trous débouchants (45) correspondant à ladite pluralité de broches (43),
- chacun desdits deux éléments tronconiques (55) ou plus comprenant un rebord circulaire proximal (53) auquel la surface conique (52) est raccordée, prévus avec une pluralité de trous débouchants (54) correspondant à ladite pluralité de broches (43),
- ledit support annulaire distal (47) étant prévu avec une pluralité de trous (48), encore plus préférablement des trous débouchants, correspondant à ladite pluralité de broches (43).

6. Dispositif d'étanchéité (24) selon l'une quelconque des revendications précédentes, **caractérisé en ce que** la membrane proximale (49) comprend un rebord circulaire (60) délimitant le trou débouchant central (62), à partir duquel un bord en saillie (61) s'étend, une extrémité proximale (59) de l'élément tronconique distal (44) étant logée dans la membrane proximale (49) en contact avec une surface distale de son rebord circulaire (60) et intérieurement par rapport à un bord en saillie (61) de cette dernière, la membrane proximale (49) ayant une forme avec un plan circulaire qui est légèrement comprimé vers l'avant le long d'un arc de cercle, de préférence de l'ordre de 90° à 120°.

7. Dispositif d'étanchéité (24) selon l'une quelconque des revendications précédentes, **caractérisé en ce qu'**une extrémité distale de la surface conique (52) d'au moins l'un parmi lesdits deux éléments tronconiques (55) ou plus de ladite membrane distale est reliée à une surface cylindrique (57).

8. Dispositif d'étanchéité (24) selon l'une quelconque des revendications précédentes, **caractérisé en ce qu'**il est logé dans un premier et un second élément annulaire (14, 15), couplés entre eux afin de former un récipient du dispositif (24) et ayant un trou débouchant central, les premier et second éléments annulaires (14, 15) étant de préférence en polycarbonate, les premier et second éléments annulaires (14, 15) étant plus préférablement collés entre eux.

9. Dispositif d'étanchéité (24) selon l'une quelconque des revendications précédentes, **caractérisé en ce que** :
- l'élément tronconique proximal (42) est réalisé avec au moins un matériau choisi dans le groupe comprenant un élastomère thermoplastique, de préférence Hytrel de DuPont® et le polycarbonate et/ou
- la membrane proximale (49) est réalisée avec au moins un matériau choisi dans le groupe comprenant la silicone, Laprène, un mélange de caoutchouc butadiène-styrène (SBR) et de caoutchouc nitrile (NBR), le matériau de la membrane proximale (49) ayant de préférence une dureté égale à 40 Shore A, et/ou
- l'élément tronconique distal (44) est réalisé à partir d'élastomère thermoplastique, de préférence Hytrel de DuPont®, et/ou le polycarbonate et/ou
- lesdits deux éléments tronconiques (55) ou plus de ladite membrane distale sont réalisés avec au moins un matériau choisi dans le groupe comprenant la silicone, Laprène, le mélange de caoutchouc butadiènestyrène (SBR) et de caoutchouc nitrile (NBR), le matériau de la membrane proximale (49) ayant de préférence une dureté égale à 40 Shore A, et/ou
- le support annulaire distal (47) est réalisé avec au moins un matériau choisi dans le groupe comprenant un polymère thermoplastique, de préférence de l'acrylonitrile butadiène styrène ou ABS, et le polycarbonate.

10. Trocart comprenant un corps (13, 14, 15, 25, 26) couplé à une canule cylindrique creuse (29) dans laquelle un obturateur (23) peut être inséré, et un capuchon supérieur (10), ayant un sommet, qui est couplé de manière amovible au corps (13, 14, 15), **caractérisé en ce que** le corps (13, 14, 15, 25, 26) comprend un dispositif d'étanchéité (24) selon l'une quelconque des revendications 1 à 9.
